# EUROPEAN PATENT APPLICATION

(11) **EP 3 181 071 A1**
(43) Date of publication of application: **21.06.2017**
(21) Application number: 15003663.0
(22) Date of filing: 23.12.2015
(51) Int. Cl.: A61B 17/34, A61B 17/00

(54) **DEVICE FOR PROVIDING INTRAPERICARDIAL ACCESS**

(30) Priority: 17.12.2015 PL 41535215
(71) Applicant: Innovations for Heart and Vessels Sp. z o.o., 40-171 Katowice (PL)
(72) Inventor: Buszman, Piotr, 40-761 Katowice (PL); Buszman, Pawel, 40-748 Katowice (PL); Milewski, Krzysztof, 40-748 Katowice (PL)
(74) Representative: Gizinska-Schohe, Malgorzata

(57) **Abstract**

The invention relates to a device for providing intrapericardial access, comprising an access channel, characterised in that it comprises: a longitudinal body **(1)** with a handle **(2)** at the proximal part, and an atraumatic distal tip **(3)** having a spiral profiling, and an inner access channel **(4)** extending along its entire length; and, placed inside the access channel **(4),** a movable dagger **(5)** having a proximal tip **(6)** and a sharp distal tip **(7);** and, optionally, a cup-shaped stabilising plate **(8)** placed on the outer surface of the body **(1),** wherein the distal tip **(3)** of the body and the distal tip **(7)** of the dagger have, at least on a part of their lengths, a conic shape which is gradually narrowing to the distal tip **(7).**

## Description

The object of the invention is a device for providing intrapericardial access, i.e. a cardiac device which enables diagnostics of the fluid in the pericardial sac and/or therapy in certain clinical situations, such as: pericardial decompression in case of the tamponade or pericarditis, therapy connected with topical administration of therapeutic substances and drugs, including also stem cells, growth factors and chemotactic factors.

A standard technique of the procedure for providing intrapericardial access (called pericardiocentesis) consists in a transdermal puncture of the pericardial sac with a special needle under local anaesthesia, controlled by one of the following techniques: ECG, transthoracic echocardiography (TTE) or fluoroscopy. Upon the pericardial puncture, a guidewire is introduced to the sac through the needle's inner lumen, followed by a special catheter.

The most common puncture site is the area below the left costal arch, near the xiphisternum, and alternative sites include the same area, but on the right side, heart apex area, and parasternal area at intercostal spaces. These alternative access sites, although important in certain clinical situations, yet they are connected with a higher risk of complications.

One of the complications of pericardiocentesis might be a discontinuity of the walls of the heart or coronary arteries, which in turn might be connected with a high risk of death. Other significant complications include the puncture of right atrium or right ventriculum, arrhythmia, aeroembolism or pneumothorax.

In many clinical cases, aspiration of the fluid or intrapericardial administration of drugs should be repeated multiple times, particularly in cases of recurrent effusion (14-50% cases) and when there is a need for multiple administration of drugs. In these cases, it is related to the higher risk of the occurrence of the above mentioned complications.

The description of US2013274782A1 patent application discloses a device for providing intrapericardial access, which comprises a tip adapted for pericardial puncture. The device with the distal tip has an inner channel. The distal portion of the inner sheath of the device comprises a helical (spiral) thread having a cutting surface adapted to cut the pericardial sac. The inner channel of the device is relatively narrow and is used, e.g., to deploy a guidewire, wherein, at the distal portion, the tip has a laterally positioned opening, so that the guidewire is not introduced axially. Moreover, the essential body of the device is one-piece.

The aim of this invention is to propose a device used for gaining intrapericardial access, which will provide safety during multiple punctures of the pericardial sac. A properly designed atraumatic tip of the device may minimise the perforation risk for the walls of the heart or coronary arteries. Assembling the device from two parts which are movable with respect to each other increases its multi-functionality.

The object of the invention is the device for providing intrapericardial access, comprising an access channel, characterised in that it comprises:
- a longitudinal body with a handle at the proximal part, and an atraumatic distal tip having a spiral profiling, and an inner access channel extending along its entire length, and,
- placed inside the access channel, a movable dagger having a proximal tip and a sharp distal tip,
- and, optionally, a cup-shaped stabilising plate placed on the outer surface of the body,
wherein the distal tip of the body and the distal tip of the dagger have, at least on a part of their lengths, a conic shape which is gradually narrowing to the distal tip.

The distal tip of the dagger has a spiral profiling. The access channel from the proximal side has an outer or inner thread. Inside the channel there is a threaded area and on the outer surface of the dagger there is a threaded area.

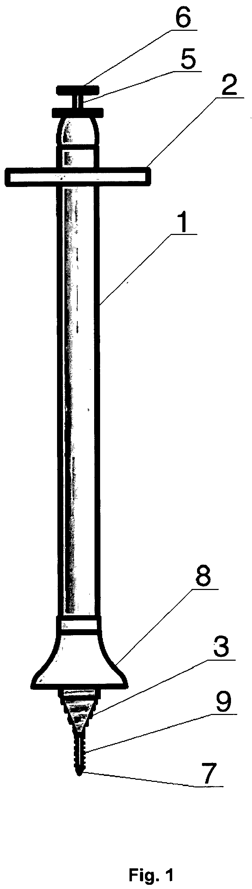

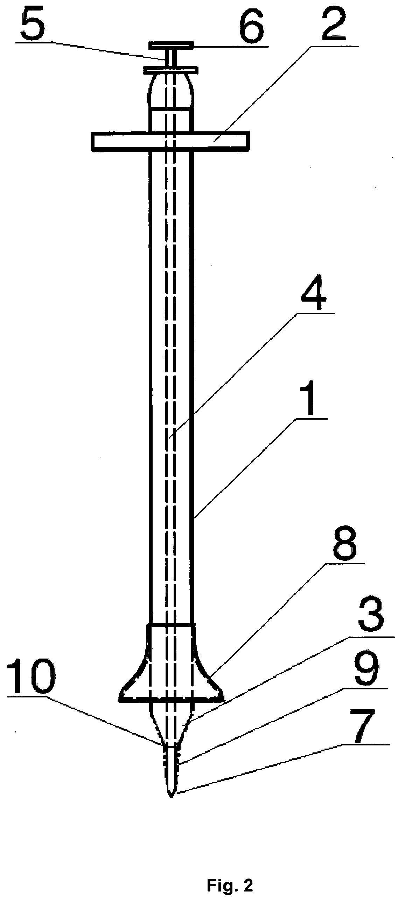

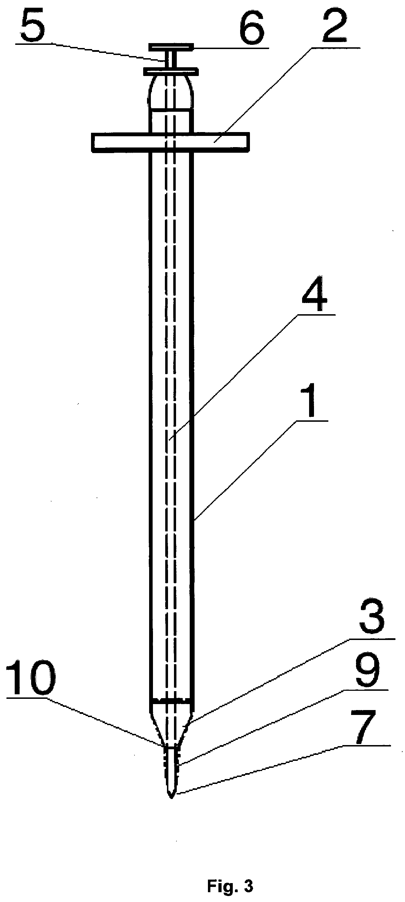

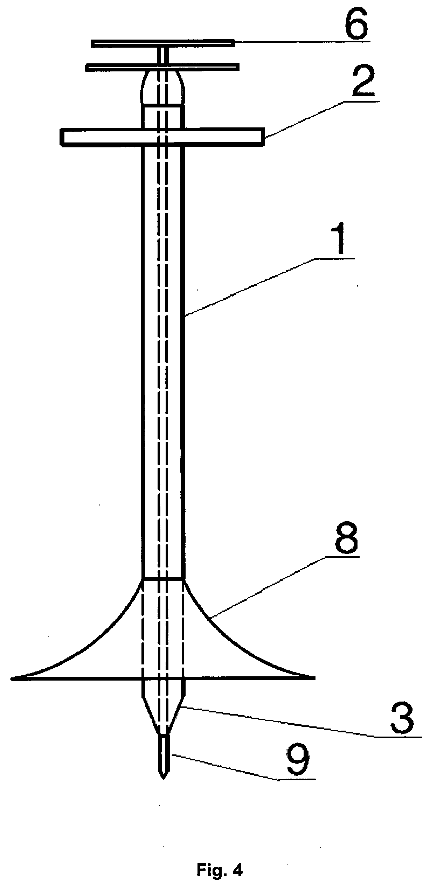

An embodiment of object of the invention is presented on a drawing, wherein fig. 1 shows a general side view of the device in a variant with the stabilising plate, fig. 2 shows a longitudinal section of the device of fig. 1, fig. 3 shows a longitudinal section of the device in a variant without the stabilising plate, and fig. 4 shows handles and the plate with wider diameter, and the distal part of the dagger without the spiral profiling.

The device for providing intrapericardial access comprising the access channel, according to the invention, consists of two basic separable parts, i.e. the first part - the longitudinal, cylindrical body **1** comprising the handle **2** at the proximal part, which facilitates the work of medical staff, and the atraumatic tip **3** at the distal part, wherein along the entire body **1,** inside of it, there is the centrally located access channel **4** with the opening **10** which ends it at the distal side; and the second part - the movable dagger **5** whose length is somewhat longer than the length of the body. The dagger is made of a solid material (does not have an opening/channel) and has the proximal tip **6** widened in relation to the dagger's diameter, which functions as a handle, as well as the sharp distal tip **7.** The dagger tip **6** can have various shapes and sizes. The sizes of the dagger's cross-section (e.g. a diameter in case of a cylindrical dagger) are adapted to the sizes of the access channel **4** and the opening **10,** so that the dagger can be introduced into and removed from this channel. It is possible for the channel **4** to be threaded inside, and then the dagger **5** is also threaded on its outer surface. In such an embodiment, moving the dagger in the body is performed by way of screwing/unscrewing. At the area of the atraumatic distal tip **3** of the body and possibly also the distal part **9** of the dagger, optionally, there is the spiral profiling (a convex threading) which enables the helical motion, i.e. screwing, of the entire device during its introduction into the pericardial space. Simultaneously, the distal tip **3** of the body and the distal part **9** of the dagger are, at least on a part of their lengths, shaped conically with the diameter narrowing to the distal side till the top sharp part of the dagger. Optionally, the funnel-like stabilising plate **8** can be placed on the body.

In contrast to the solutions known in the state of art, the device according to the invention proposes a two-part construction, i.e. the device consists of the outer part - the body - and the movable (separable) dagger placed in the access channel of the device and, if there is a need, removed from the channel. The distal tip of the body contains the opening which is a part of the access channel through which it is possible to introduce other medical devices to the pericardial space, as well as provide fluid or suspension drugs, medical substances etc. The access channel has a relatively large diameter of 0.5 mm to 5 mm, e.g. from 1 to 5 mm. Moreover, through the channel the fluid from the pericardial sac can be aspirated. This enables performing multiple-step medical procedures, using different devices introduced successively into the access channel of the device according to the invention.

The device uses the dagger of a unique design and its cooperation with the specially designed threaded and conic tip of the body, what gives easy, safe and predictable motion of the device in the direction of the pericardial sac. The proximal tips of the device, both of the body and the dagger, function as handles for medical staff and they can have various forms, sizes or shapes, what will be apparent for a person skilled in the art. Optionally, it is predicted for the device to use additional funnel-like outer part in a form of the plate stabilising the device while gaining intrapericardial access. The plate in a form of a collar surrounds the device's body, and the widened cup-shaped form is turned to the side of a patient to support the suitable position of the device during the performed procedure. The cup-shaped collar of the stabilising plate can have various sizes (various diameters). The plate has a central hole which enables its placement on the device's body. Preferably, the plate is adjusted in a slidable way with a certain resistance, so that it is possible to slide it freely depending on the anatomical conditions during the procedure, but so that after the adjustment it stays on the set position of the body's length.

The distal tip of the body together with the distal tip of the dagger have, at least partially, a conic shape which gradually narrows to the side of the distal end of the device, thereby forming a mild form adjusted to move the device through tissues and the pericardial sac to the pericardial space. Preferably, the distal tip of the body has the spiral profiling (helical threading) and possibly also the distal tip of the dagger has such a spiral profiling.

During the conduction of medical procedures, the dagger with threaded or non-threaded outer part is used to puncture tissues. Upon the tissue puncture, the dagger is removed (or unscrewed), at the same time screwing the body deeper into tissues. If the resistance occurs, while screwing the body, it is possible to re-introduce the dagger with the sharp tip which helps to overcome the resistance.

During the conduction the procedure using the device according to the invention, gaining the access and the fact of the device's tip reaching the pericardial cavity can be confirmed in several ways: (a) by the aspiration of the fluid through a syringe attached to the proximal part of the device, (b) by the occurrence of ST-segment elevation thanks to the electrocardiograph (ECG) attached to the proximal part of the device, c) by introducing and monitoring the position of the guidewire in the pericardial cavity using fluoroscopy, (d) by confirming the position of the device's tip and the guidewire by the ultrasonography test.

Upon confirmation of gaining intrapericardial access in a proper way, it is possible to permanently remove the dagger and thereby obtain the inner lumen of a relatively large diameter, enabling the aspiration of the fluid or administration of drugs, stem cells etc.

The distal part of the body can be covered with a soft hydrophilic material. At the distal top of the body there is the opening with a diameter which enables the introduction of a dagger, guidewire or catheter. The continuation of the opening is the channel which runs through the entire length of the device. Proximally, the channel can end with a thread to which a syringe, line or plug can be screwed.

The device according to the invention can be made of various known materials accepted in this type of applications, for which no negative effects are observed when interacting with tissues and blood, e.g. of biocompatible plastics, flexible Teflon, fragments of titanium, stainless steel 316L, cobalt-chrome alloy L605.

Reference numerals on drawings:
- 1.: body
- 2.: body handle
- 3.: atraumatic distal tip of the body
- 4.: access channel
- 5.: dagger
- 6.: proximal tip of the dagger
- 7.: sharp distal tip of the dagger
- 8.: stabilising plate
- 9.: distal part of the dagger
- 10.: opening which ends the access channel of the body

## Claims

1. A device for providing intrapericardial access, comprising an access channel, **characterised in that** it comprises
- a longitudinal body (**1**) provided with a handle (**2**) at the proximal part, and an atraumatic distal tip (**3**) having a spiral profiling, and an inner access channel (**4**) extending along its entire length, and,
- placed inside the access channel (**4**), a movable dagger (**5**) having a proximal tip (**6**) and a sharp distal tip (**7**),
- and, optionally, a cup-shaped stabilising plate (**8**) placed on the outer surface of the body (**1**),
wherein the distal tip (**3**) of the body and the distal tip (**7**) of the dagger have, at least on a part of their lengths, a conic shape which is gradually narrowing to the distal tip (**7**).

2. The device according to claim 1, **characterised in that** the distal tip (**7**) of the dagger has a spiral profiling.

3. The device according to claim 1 or 2, **characterised in that** the access channel (**4**) from the proximal side has an outer or inner thread.

4. The device according to claim 1 or 2, **characterised in that** inside the channel (**4**) there is a threaded area and on the outer surface of the dagger (**5**) there is a threaded area.
